# EUROPEAN PATENT APPLICATION

(11) **EP 1 336 658 A1**
(43) Date of publication of application: **20.08.2003**
(21) Application number: 02290389.2
(22) Date of filing: 18.02.2002
(51) Int. Cl.: C12N 15/82, C12Q 1/68, A01H 5/00

(54) **G1/S phase specific, S phase specific and meristem specific transcription control element in a transcribed region**

(71) Applicant: CENTRE NATIONAL DE LA RECHERCHE SCIENTIFIQUE DAE, 75794 Paris Cédex 16 (FR)
(72) Inventor: Chabouté, Marie Edith, 67800 Bischheim (FR); Hatzfield, Yves, 59000 Lille (FR); Reuzeau, Christophe, 24350 Saint-Apre (FR); Schoonjans, Reinhilde, 9031 Drongen (BE)
(74) Representative: De Clercq, Ann

(57) **Abstract**

The present invention relates to a new identified transcription control element for induction of expression in the G1/S transition phase of the cell cycle, for S phase specific expression and meristem specific expression and the uses thereof. The control element is an E2F box located in the transcribed region of a gene. More particularly the E2F box of the tobacco RNR1b gene was identified and shown to be located in the 5'UTR of the gene. From that position it is responsible for the specific expression of the gene in the G1/S phase, the S phase and in meristem cells. By using the control element in the 5'UTR the invention thus offers a new tool to influence the activity of promoters, to control the expression of a gene of interest. The invention further relates to nucleic acids, vector constructs, host cells and methods to use the invention.

## Description

### Field of the invention

The present invention relates to the field of plant molecular biology and more particularly, the present invention describes the use of a transcriptional control element found in the transcribed region of the RNR1b gene of tobacco to control G1/S induction, and/or S phase specific and/or meristem specific gene expression in plant cells.

### Background to the invention

Ribonucleotide reductase is a key enzyme in the pathway of DNA synthesis. It catalyzes the reduction of ribonucleosides diphosphates (NDPs) into deoxyribonucleosides diphosphates (dNDPs), a rate-limiting step in DNA synthesis (Reichard, 1988). In both mammals and plants, ribonucleotide reductases belong to the RNR Class I in which the active enzyme consists of two large (R1) and two small (R2) subunits encoded by RNR1 and RNR2 genes, respectively. In earlier studies the inventors cloned three cDNA's encoding a tobacco ribonucleotase reductase: one cDNA encoding the small subunit (RNR2) and two cDNA's encoding the large subunit (RNR1a and RNR1b). The cDNA's were isolated from BY2 tobacco cell culture, that are highly synchronizable (Chabouté et al, 1998).

Progression of the cell cycle is associated with the phase-specific transcription of genes encoding proteins that control the cell cycle or that are involved in DNA replication. These genes are primarily regulated at the transcriptional level during the different stages of the cell cycle. For the RNR2 gene it is shown that two E2F control elements are present in the promoter region , that those elements specifically interact with a tobacco E2F factor and that they mediate up-regulation of the promoter at the G1/S transition of the cell cycle (Chabouté et al, 2000). Up to now, only a few E2F target genes which have an E2F binding site (consensus sequence TTTC/GC/GCGC) in their promoter, have been characterized in plants. Recently, there is evidence that Arabidopsis E2F1 factor binds an E2F control element in the promoter of an S phase-regulated gene AtCDC6 (De Jager et al., 2001). Also, potential E2F binding sites, found in the promoter of the rice proliferating cell nuclear antigen (PCNA), mediate transcriptional activation in actively dividing cells and tissues of tobacco (Kosugi et al, 2002). Recently, E2F cis- elements were also identified in the tobacco PCNA promoter and one of these elements was shown to act essentially as a strong transcriptional repressor of the PCNA gene in differentiated tissue (Egelkrout et al, 2001).

Since RNR1b gene shows a very specific cell-cycle regulated expression pattern, it is the aim of the present invention to determine the elements responsible for that specific expression, to isolate them and use them for the purpose of obtaining regulated gene expression.

### Summmary of the invention

The present invention offers a new control element which controls the meristem specific expression and/or G1/S phase induction and/or S-phase specific expression of the RNR1b gene of tobacco. Surprisingly the inventors found that this control element was situated in the transcribed region of the RNR1b gene. After further investigation the inventors found that the control element responsible for this specific gene expression is an E2F control element, more particularly an E2F element present in the 5'UTR of the RNR1b gene.

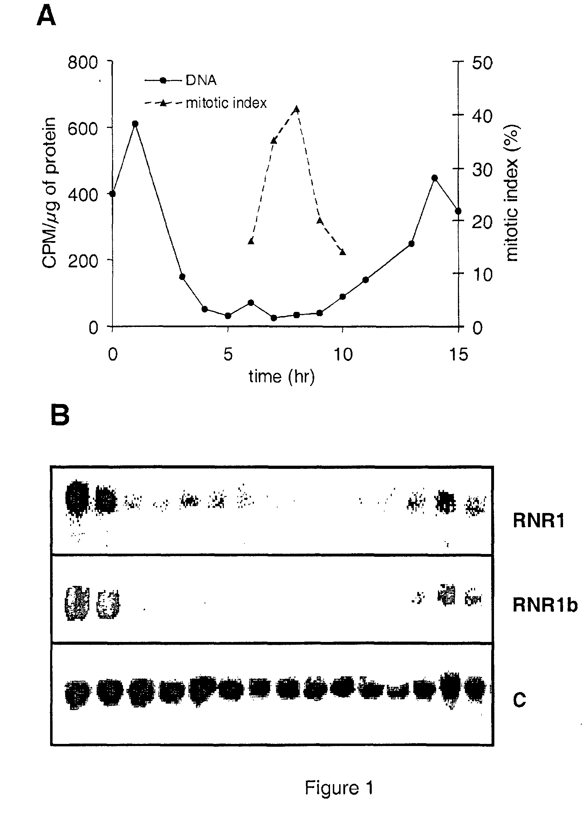

The expression of RNR genes is tightly linked to the S phase of the cell cycle were DNA replication takes place. By using probes specific for the 3'UTR's of the two RNR1 genes (RNR1a and RNR1b) it was observed for the first time that RNR1b is exclusively expressed during the S phase in BY2 synchronized tobacco cells (figure 1), whereas RNR1a gene is also expressed outside the S phase, notably at the G2/M transition. The RNR1b upstream activator sequence (UAS) was cloned, sequenced and analyzed. The high degree of G1/S phase induction and S phase specificity of the RNR1b UAS is a unique feature of the control element of the present invention and offers the person skilled in the art a unique tool to mediate S-phase specific gene control and induction of the gene expression early in the S phase i.e. at the G1/S transition phase.

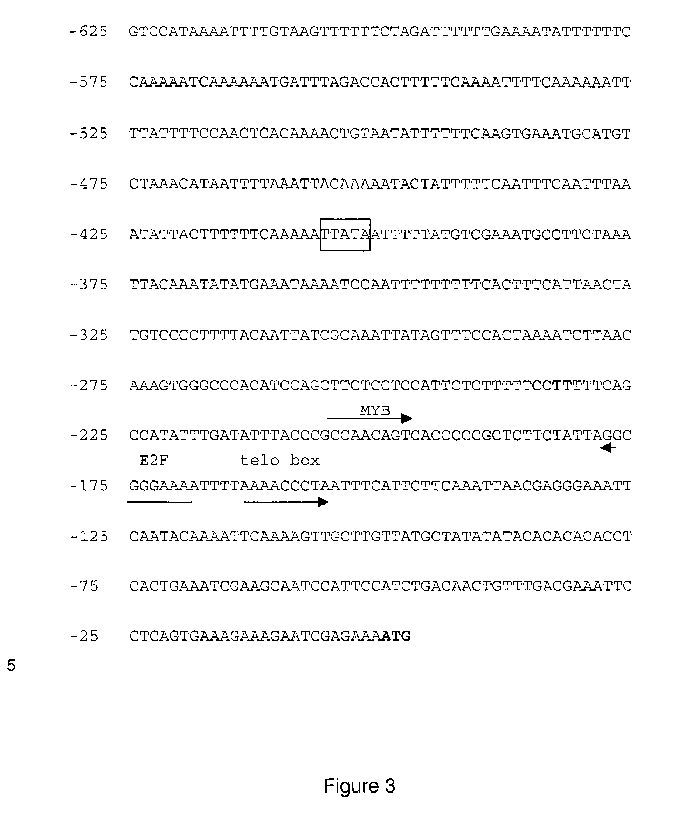

The particular region in the RNR1b UAS responsible for the G1/S induction and S phase specificity was identified in the present invention by mutations in the UAS that resulted in disruption of the G1/S induction and S phase specific gene expression. The sequence, which is responsible for the G1/S induction and S phase specific gene expression in the UAS is situated downstream of the putative TATA box, and surprisingly, it is found to be positioned downstream of the transcription initiation site (figure 3). The newly identified regulatory element is part of the 5' UTR which is (partially) published in the database as part of the RNR1b transcript (database accession number Y10862).

Interestingly, this regulatory element was found to be a unique E2F element in the reverse orientation.

This is the first time that it is demonstrated (in plants) that G1/S induction and S phase specific gene expression is mediated through an E2F control element from a position in the 5'UTR of a gene rather than in the promoter of the gene.

Using reporter gene analysis in synchronized BY2 cells, it was demonstrated that the WT tobacco RNR1b UAS activity is only detected at the entry of the S phase (i.e. the G1/S transition) to reach a maximal induction level in the S phase. In addition, a meristematic specific activity of the RNR1 b UAS is detected in plantlets using a GUS reporter gene. Mutation of the E2F control element abolishes both the G1/S and S-phase activity and meristem activity of the RNR1b UAS. Thus the single E2F control element in the tobacco RNR1 gene is essential to drive G1/S induction and S phase specific gene expression and the meristematic activity in plants, indicating that the E2F control element plays an important role in the induction of the RNR1b gene during both plant cell cycle progression and development.

Accordingly, the present invention relates to a new gene expression control element, which is an E2F control element present in the transcribed region of a gene. The invention further relates to the nucleic acids, vectors, host cells and host organisms containing that (isolated) novel control element. Also the present invention relates to a new method for mediating G1/S induction and S phase specific gene expression and/or meristem specific gene expression based on the use of this new gene control element in the context of the transcribed region.

### Detailed Description of the Invention

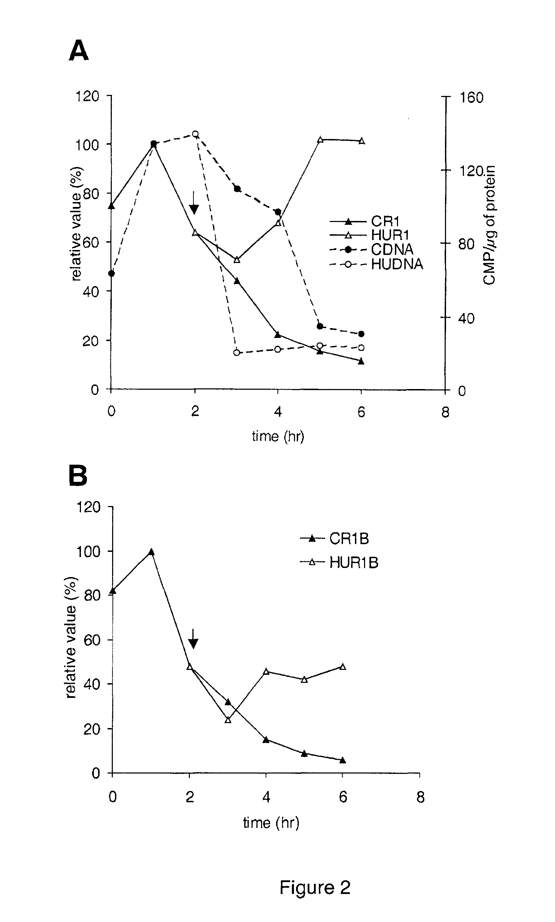

In a previous report, the inventors identified two RNR1 cDNAs and one RNR2 cDNA (Chabouté et al, 1998). Since the coding regions of both RNR1 cDNAs are closely related, 3'-specific probes were necessary to distinguish the expression pattern of each cDNA. Interestingly, RNR1b expression is induced at the G1/S transition and is restricted to the S phase of the cell cycle (example 1, figure 1) whereas overall RNR1 gene expression is detected both in S phase and at the G2/M transition, suggesting there that the two RNR1 genes are differently regulated during the cell cycle. When DNA replication is blocked in S phase (example 2, figure 2), after RNR gene expression has been induced, the RNR1b mRNA level is stabilized. In contrast, global RNR1 transcript level sharply increases, suggesting that the response is mainly due to the induction of the RNR1a gene. These results indicate that the two RNR1 genes are regulated by different pathways, leading to a different cell-cycle expression.

Transcriptional regulation of the RNR1b gene has revealed several interesting features. The RNR1b UAS sequence is the first to be cloned in plants and unlike its animal counterparts, it contains one putative E2F binding site, TTTCCCGC, located at - 177 bp from ATG (example 3, figure 3).

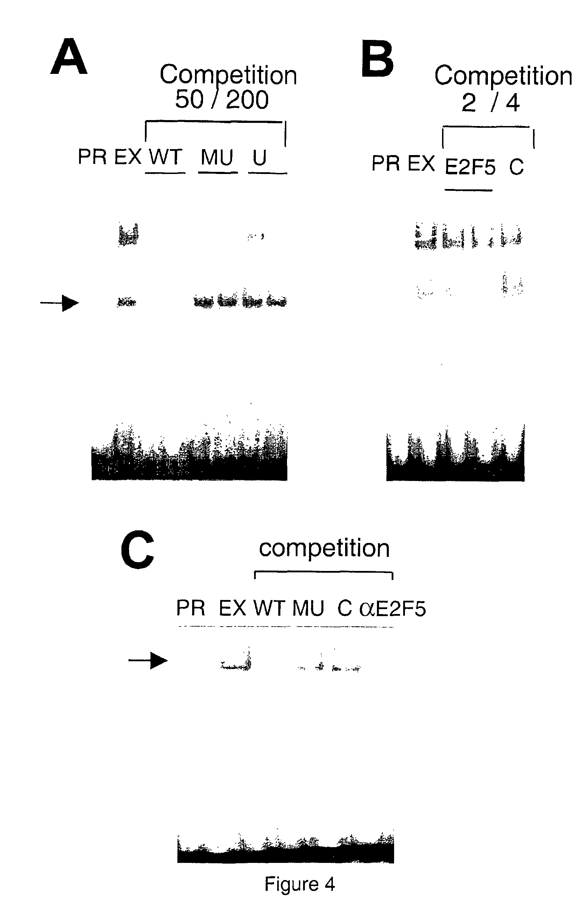

The inventors show by gel shift assays (example 4, figure 4) that a specific nuclear complex found in exponentionally growing cells associates with the E2F site in the RNR1b UAS. In addition, a purified tobacco E2F factor interacts with the E2F binding site.

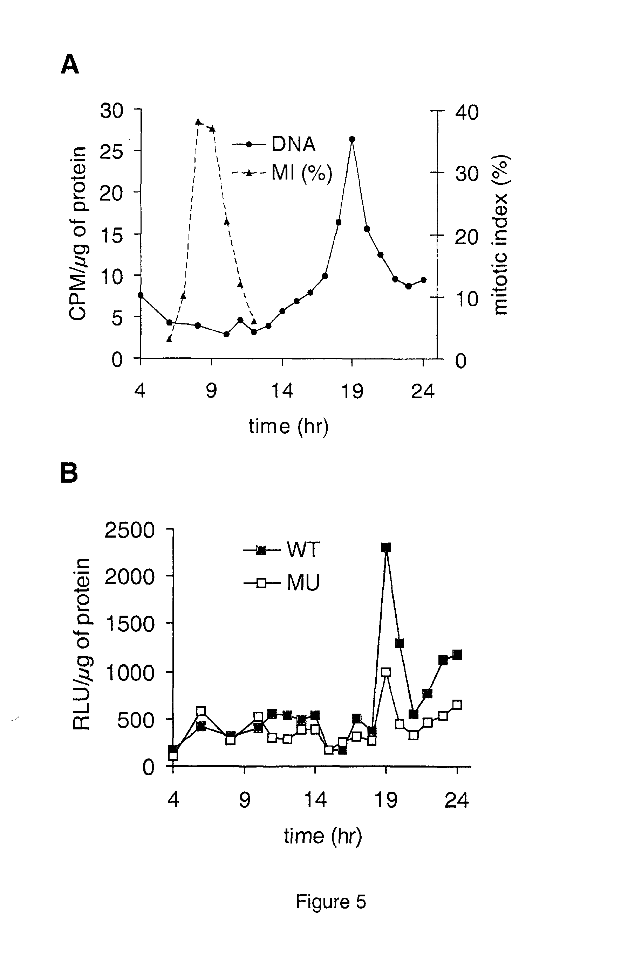

Functional analysis of the role of the E2F control element on the activity of the RNR1b UAS was investigated by fusing either the wild type (WT) UAS to the luciferase gene or by fusing the UAS with a mutation in the E2F control element to a reporter gene. The reporter gene expression experiments (example 5, figures 5) showed that RNR1b gene is specifically induced at the G1/S transition of the cell cycle with a maximum in S phase, whereas the mutated UAS showed only a faint induction. The E2F element is directly involved in RNR gene induction and acts as transcription activator of the tobacco RNR1b gene.

When fused to the GUS reporter gene, RNR1b UAS activity is shown to be restricted to meristematic tissues in developing plantlets, and, in particular, to the axillary meristems (lateral roots and axillary buds) but no activity was detected in the apical meristem. Conversely, in plant histone promoters, octamer and nonamer elements are responsible for S-phase induction (Taoka et al, 1999) and lead to strong GUS expression in root tips and apical meristems of growing plantlets (Chaubet et al, 1996). No GUS activity was detected in the transgenic seedlings harboring the mutated-E2F construct, suggesting that E2F element drives meristematic activity of the RNR1b UAS, especially in the axillary meristems. These results emphasize the importance of the E2F control element in the G1/S phase-specific induction of the RNR1b gene.

In conclusion, the invention demonstrates for the first time in plants, that a single E2F site is involved in both cell cycle (G1/S induction and S phase activity) and plant developmental regulation (meristem activity). Moreover, it is demonstrated for the first time that a functional E2F element controlling the expression of the plant gene is present in the transcribed region of the gene. This E2F control element was further isolated and characterized.

Accordingly, the present invention embodies the use of an E2F control element to obtain G1/S-phase and/or S-phase and/or meristem-specific gene expression in plants, characterized in that said E2F control element is present in a transcribed region of a gene of interest.

In another embodiment of the invention, the E2F control element to be used as mentioned above is present in a 5' UTR of a gene of interest.

The inventors also identified another cis-element in the RNR1b UAS which was identified as a putative Telobox. It is possible that the Telobox plays a role in E2F-mediated regulation of the RNR1b UAS. The sequence of the Telobox of the RNR1b gene is represented in SEQ ID NO 8 and is as follows: 5' AAAACCCTA-3'Therefore the present invention embodies the use of an E2F control element as mentioned above, wherein said E2F control element is combined with a Telobox sequence.

The combination of the two control elements can be established by operable linking them in the same genetic construct.

Another embodiment of the invention is the use of an E2F control element as mentioned above, wherein said E2F control element is present in a 5' UTR of a plant RNR1b gene. For example, said E2F control element is comprised within the sequence as given in SEQ ID NO 1.

Within the scope of the invention is the use of an E2F element to regulate the expression of a gene of interest. Expression of a gene is commonly driven by a promoter sequence and therefore, the present invention further embodies the use of an E2F control element as described above, wherein said transcribed region of said gene of interest is preceded by a promoter

The inventors also identified another cis-element in the RNR1b UAS which was identified as a MYB like control element. It is possible that the MYB-like element play a role in E2F-mediated regulation of the RNR1b UAS. MYB binding site was shown to drive M phase-specific transcription of B-type cyclin gene in tobacco (Ito et al, 1998). The MYB box of the RNR1b UAS of the present invention has the sequence GCCAACAGT and is further referred to as SEQ ID NO 9.

According to this aspect of the invention, the invention embodies the use of an E2F control element as described above, wherein said transcribed region is combined with a MYB box.

In a more particular embodiment, the combined MYB box as described above is present in a promoter, or is present in the 5'UTR.

More particularly, the invention embodies the use of an E2F control element as described above, wherein said transcribed region of a gene is combined with the promoter of a plant RNR1b gene

A further embodiment of the invention is the use of an E2F control element as described above, wherein said transcribed region of said gene of interest is preceded by a sequence as given in SEQ ID NO 2

The inventors isolated and characterized the UAS sequence of the tobacco RNR1b gene. Accordingly, the present invention embodies an isolated nucleic acid, comprising an upstream activator sequence comprising:
(a) at least one promoter sequence or a functional part thereof, and
(b) at least one 5' UTR or a functional part thereof comprising at least one E2F control element.

In a related embodiment of the invention, the promoter contains at least one TATA box and at least one MYB box and the 5'UTR as mentioned above contains at least one Telobox.

A further embodiment of the invention is an isolated nucleic acid as described above comprising a sequence selected from the group consisting of:
(a) a nucleic acid comprising the DNA sequence as given in SEQ ID NO 2 or 3 , or the complement thereof,
(b) a nucleic acid specifically hybridizing with the nucleic acid as defined in (a)
(c) a nucleic acid which is diverging, due to the differences between alleles, to a nucleotide sequence as defined in (a) or (b), and
(d) a nucleic acid consisting of the sequence as given in any of SEQ ID NOs 1, 2 or 3.

In one embodiment, said sequence (a) to (d) is an upstream activating sequence. Another embodiment of the invention is an isolated nucleic acid comprising in the 5' to 3' direction in the following order at least one TATA box, at least one MYB box, at least one E2F control element in the sense or reverse orientation, and at least one Telobox. In a more particular embodiment there is a transcription initiation site between the MYB box and the E2F element.

Regulation of expression via E2F control elements is very complex. E2F elements can occur in multiple copies in the promoter of a gene and they might have each a separate function for the control on expression of the gene. Also the orientation and the sequence of the E2F element play a role in this control. Furthermore, it is shown in the present invention that also the position of the E2F element is an important factor in the functionality of the control element.

Therefore within the scope of the invention is the use of E2F elements in one or more copies in the sense or in the reverse orientation and/or in a defined position situated in the transcribed region of a gene.

In a particular embodiment of the present invention, the isolated nucleic acid as mentioned above comprises in the 5' to 3' direction the following elements derived from a gene, for instance a plant gene, for instance a plant RNR1b gene: at least one TATA box, at least one MYB box, at least one E2F element and at least one Telobox. In a further embodiment the isolated nucleic acid as mentioned above comprises in the 5' to 3' direction at least one TATA box, at least one sequence as represented in SEQ ID NO 9, at least one sequence as represented in SEQ ID NO 4 and at least one sequence as represented in SEQ ID NO 8. In a more particular embodiment there is a transcription initiation site between the MYB box or the sequence as represented in SEQ ID NO 9 and the E2F element or the sequence as represented in SEQ ID NO 4.

In a particular embodiment of the invention, the control element E2F of the present invention is combined with other control elements such as control elements responsive to hormones. Examples of hormones are, but are not limited to giberrillin, auxin, cytokine, ethylene, brassinosteroid etc. In a particular example, the E2F control element is combined with an auxin responsive element. Auxin is a plant hormone that is involved in the regulation of the cell cycle via the mechanism of auxin-induced or auxin-responsive genes. Many transcription factors are responsive to auxin and when activated they can mediate the expression of other auxin induced genes.

Accordingly the present invention also embodies an isolated nucleic acid as described above wherein the E2F control element is combined with at least one hormone responsive element such as an auxin responsive element.

In a particular embodiment of the invention the isolated nucleic acids as described above are cDNA or genomic DNA.

In a further embodiment, the invention relates to a vector construct comprising at least one isolated nucleic acid as described above.

Also in a further embodiment the invention relates to a vector, comprising at least one nucleci acid as describedabove, wherein said vector is an expression vector and wherein said isolated nucleic acid is operably inserted upstream of a gene of interest.

Furthermore, the invention embodies a host cell comprising an isolated nucleic acid or a vector construct as described above. Said host cell can be an eukaryotic or a prokaryotic cell, for instance a bacterial, insect, fungal, yeast, plant, animal or mammalian cell.

In the present application it is described for the first time that an activating E2F control element is found entirely in a 5'UTR of a plant gene, and that from that position it is responsible for meristem and/or G1/S phase induction and/or S phase specific expression of that gene. Consequently, the tobacco RNR1b upstream activating sequence (UAS) constitutes a novel tool for mediating G1/S induction and/or S phase specific gene expression and/ or meristem specific gene expression in plant cells, whose induction is mediated by an E2F factor.

Therefor another embodiment of the present invention is the use of a nucleic acid or vector construct as mentioned above for G1/S phase specific induction and/or S phase specific expression and/ or meristem specific expression of a gene of interest

A few S phase specific control elements are available in plants, such as the histone promoters. However the histone gene expression is induced one hour later than the RNR gene expression at the entry of the S phase (Chabouté et al., 1998). Moreover, the S phase induction of the plant histone genes involves OCE elements (octameric composite elements) (Taoka et al., 1999). In plants only a few data are available on cis-elements and transcription factors involved in S phase specific gene induction. PCNA gene expression is also restricted to the S phase and specific IIa and IIb elements in the promoter are controlling this expression in rice cells (Kosugi et al, 1997).

Accordingly, the control mechanism of the present invention can be used as a marker specific for G1/S and/or S phase expression, and is useful in different studies on the plant cell cycle. For example, the UAS of the present invention can be fused to a destabilized GFP and thus constitute a marker of the G1/S phase induction and S phase expression.

Therefore another embodiment of the invention is the use of any of the nucleic acids or vector constructs described herein for monitoring the expression of a gene in the G1/S transition phase, in the S phase or in the meristem, or for monitoring the endoreduplication state of the cell. Preferably said gene is easy to be monitored and/or is a reporter gene. Examples of suitable reporter genes are (but are not limited to) GUS, GFP, luciferase etc.

Endoreduplication occurs at moments wherein the G1/S transition and/or the S phase are overstimulated and are not followed by a mitosis or cytokinesis step. The invention thus also relates to the use of the E2F element as mentioned above as a marker or as indicative for the endoreduplication state of a plant cell.

In another application, the E2F control element of the invention is used to drive the ectopic expression of genes.

In a particular application, the E2F control element of the present invention is used to control the expression of an E2F factor (example 7) or an E2F factor and a DP gene (example 8) in order to establish a feedback loop to the control of the gene expression by the expressed gene itself.

DP is known to be the "dimerization partner of" E2F. The E2F/DP heterodimeric transcription factor is involved in the regulation of the G1/S transition and has been characterized in animals and more recently in plants. DP-related genes have recently been isolated from Arabidopisis (Magyar et al, 2000) and may be use in the methods of the present invention. To have expression of the E2F gene and the DP gene in the same cell, the procedures can be followed as described in example 8. As described, there are different alternatives to control the expression of both genes with the E2F control element of the present invention. Alternatively, only one of both genes, thus the E2F gene or the DP gene, can be controlled by the E2F control element of the present invention and the other gene, thus the DP or E2F gene respectively, can be controlled in a different manner.

Therefore another embodiment of the present invention is the use of a nucleic acid or vector construct as mentioned above to drive expression of an E2F gene.

Furthermore, the invention embodies the use of a nucleic acid or vector as mentioned above to drive expression of an E2F gene and of a DP gene

In a particular embodiment of the present invention, the nucleic acid or vector constructs as mentioned above are used to drive over-expression of an E2F gene or to drive overexpression of an E2F gene and a DP gene.

In a particular embodiment the invention relates to the use of a nucleic acid or vector construct as mentioned above to drive expression of a gene of interest.

Particular genes of interest are exemplified hereunder, but are not limited thereto. A particular group of genes of interest are genes, which are involved in endoreduplication. Endoreduplication can be achieved when the G1/S transition or S phase is overstimulated or continuously activated, without a subsequent completion of the cell cycle via mitosis and cytokinesis. Therefore, a control element that directs ectopic expression of genes involved in these cell cycle phases is most useful to enhance the endoreduplication effect, without having mis-expression of these genes in other parts of the plant or in other cell cycle phases. The present invention provides such a control element, namely an E2F present in a 5'UTR or transcribed region.

Also genes that encode growth factors or that encode cyclin D's are interesting genes to put under the control of the E2F element of the invention, since these genes are most active in the meristem and/or during the G1/S transition or S phase of the cell cycle.

All cell cycle genes and especially genes that are involved in the G1/S transition or S phase of the cell cycle are interesting genes to put under the control of the UAS of the present invention. Specific examples of such genes are "cell division cycle" genes such as cdc6 and cdc27, "origin of replication complex" genes such as ORC and "inhibitor of cyclin-dependent kinase" genes such as ICK's. Also interacting partners of these G1/S or S phase specific genes and proteins are preferred candidates to put under the control of the UAS of the present invention.

Another group of genes of interest to put under control of the E2F element of the present invention or that can be combined with the E2F element of the present invention, are genes that are responsive to hormones. In a particular application the UAS of the invention is used to control the expression of auxin responsive or auxin induced genes. In another example, the UAS of the invention will be used to drive the expression of a gene of interest in combination with the expression of an auxin responsive or an auxin induced gene.

Accordingly in another embodiment, the invention relates to the use of a nucleic acid or vector construct as mentioned above to drive expression of hormone induced or hormone responsive gene, such as an auxin induced or auxin responsive gene. Often hormone induced genes are involved in growth regularion and therefore the present invention further refers to the use of a nucleic acid or vector construct as mentioned above to drive expression of a growth regulatory gene.

Furthermore, the invention relates to the use of a nucleic acid or vector construct as mentioned above to drive expression of a gene of interest in combination with the expression of a hormone induced or hormone responsive gene, such as an auxin induced or auxin responsive gene.

Other useful applications of the invention are described in the examples and result in the embodiments as described hereunder.

The invention further relates to the use of an E2F control element located in the 5'UTR or transcribed region as described above, to construct an expression cassette that is active in the G1/S transition phase and/or in the S phase and/or in meristematic cells.

In another embodiment, the invention relates to the use of the E2F control element in a 5' UTR or transcribed region as described above, to construct a hybrid gene that is expressed in the G1/S transition phase and/or the S phase and/or in meristematic cells.

In a related embodiment the invention relates to the use of an E2F control element in a 5'UTR or as described above in the transcribed region of a gene, in multiple copies to obtain improved expression and/or improved regulation of the gene of interest.

In more particular embodiment of the invention the E2F element is placed within the 5'UTR or the transcribed region of a cell cycle gene, or a G1/S induced gene, or a S phase specific gene, or in the transcribed region of a gene already controlled by an E2F control element to enhance the expression or to enhance the regulation of that gene.

Accordingly, the invention also relates to a method for enhancing the expression of a gene of interest comprising the use of at least one E2F control element in a 5'UTR or a transcribed region of that gene of interest.

Also, the invention relates to the use of at least one E2F control element in an 5'UTR or a transcribed region of a gene for enhancing the expression of a gene of interest.

Another application of the present invention is to use at least one E2F control element in the 5'UTR or transcribed region as described above to construct a chimeric promoter which can drive simultaneous expression of two genes and which has (at least for one gene) G1/S activity and/or or S phase activity and/or meristem activity.

Therefore another embodiment of the present invention is the use of an E2F control element in a 5'UTR or in a transcribed region of a gene as described earlier, to construct a bi-directional promoter that is active in the G1/S transition phase and/or in the S phase and/or in meristematic cells .

In any of the above embodiments it might be useful to combine the E2F element of the present invention with other control elements, such as a promoter, a TATA box, a telebox or a MYB box. In a particular useful application of the invention the E2F control element is combined with a hormone responsive element, such an auxin responsive element. Therefor the present invention further embodies the use of an E2F control element in a 5'UTR as described earlier in combination with a hormone responsive element, such as an auxine responsive element.

The invention further relates to a method for the production of transgenic plants comprising introducing into a plant cell, at least one nucleic acid or at least one vector construct as described above.

The invention further embodies a method for ectopic expression of a E2F gene in a plant cell comprising:
(a) operably linking the upstream activator sequence of an RNR1b gene to an E2F gene in a nucleic acid construct, and
(b) introducing said nucleic acid construct into a plant cell.

The invention further embodies a method for ectopic expression of a E2F gene and a DP gene in a plant cell comprising:
(a) operably linking the upstream activator sequence of an RNR1b gene to an E2F gene and to a DP gene in one or several nucleic acid constructs, and
(b) introducing said nucleic acid construct or constructs into a plant cell

The invention further embodies a method for ectopic expression of a hormone regulated gene, such as an auxin regulated gene, in a plant cell comprising:
(a) operably linking the upstream activator sequence of an RNR1b gene to a hormone regulated gene, such as an auxin regulated gene in a nucleic acid construct, and
(b) introducing said nucleic acid construct(s) into a plant cell

The invention further embodies a method for ectopic expression of a gene of interest in a plant cell comprising:
1. operably linking the upstream activator sequence of an RNR1b gene to a gene of interest in a nucleic acid construct, and
2. introducing said nucleic acid construct(s) into a plant cell

The invention further embodies a method as mentioned above further comprising regenerating a plant from said plant cell.

"Upstream activator sequences" or "UAS" as used in the methods as described above, also includes the use of the 5' untranslated region or 5'UTR of the UAS.

The invention further embodies a method for building an expression cassette that is active in the G1/S transition phase and/or in the S phase and/or in meristematic cells comprising operably linking an E2F element located in a 5' UTR or in a transcribed region of a gene as described earlier, to a promoter

The invention further embodies a method for improving the expression of a gene of interest in the G1/S transition phase and/or in the S phase and/or in meristematic cells, and/or improving the regulation of a gene of interest in the G1/S transition phase and/or in the S phase and/or in meristematic cells, said method comprising operably linking an E2F element located in a transcribed region of a gene as described earlier, to said gene of interest.

In a particular embodiment of the invention these genes of interest are cell cycle genes and/or endoredupliaction genes, which might already be regulated by an E2F box

The invention further embodies a method for building a bi-directional promoter that is active in the G1/S transition phase and/or in the S phase and/or in meristematic cells, said method comprising operably linking at least one E2F element located in a 5'UTR or in a transcribed region of a gene as described earlier, to a promoter.

The methods as described above are particularly interesting to alter the plant morphology, plant phenotype and/or biochemistry and/or architecture and/or physiology of a plant and therefore the present invention also embodies transgenic plants obtainable by any of the methods as mentioned above.

Furthermore, the invention embodies a harvestable part of a such a plant, or a harvestable part of a plant which is selected from the group consisting of seeds, leaves, fruits, stem cultures, rhizomes and bulbs.

Also the progeny derived from any of these plants or plant parts is in the scope of the present invention.

It will be clear that the invention may be practiced otherwise than as particularly described in the foregoing description and examples. Numerous modifications and variations of the present invention are possible in the light of the above teaching and, therefore, are within the scope of the appended claims.

It is in the scope of the present invention to alter the expression of any gene of interest (e.g. a gene originating from a plant, a bacterium, a yeast, an animal, a fungus etc.).

G1/S phase means the transition of the G1 phase to the S phase of the cell cycle. Particularly in the present invention the E2F control element is activated early in the S phase and therefore the expression G1/S induction is used. Furthermore it is clear that the maximal activity of the RNR1b UAS of the invention is found in the S phase and therefore the inventors also use the expression of S-phase specificity or S-phase activity.

Since the G1/S transition and S phase occur in the cell cycle, the expression "to mediate G1/S induction and S phase specific expression" means to induce expression in dividing cells (in meristematic tissue and non-meristematic tissue), in endoreduplicating cells, in cells undergoing DNA duplication, in cells undergoing DNA synthesis or in cells undergoing DNA repair.

The expression "to mediate G1/S induction and/or S phase specific expression" is not strictly limited to an exclusive expression during the G1/S transition and/or in the S phase. However in some cases this expression is used for "exclusive" expression only in the G1/S transition phase and/or S phase of the cell cycle.

Similarly, the expression "to mediate meristem specific expression" is not strictly limited but can include "exclusive" expression in the meristem.

The transcribed region of a gene is the part of a gene starting from the transcription initiation site, which generally is the 5'UTR in the UAS, followed by the coding region of the gene and the 3' UTR.

The expressions "upstream activator sequence" or "UAS" of a gene relate to the 5' part of a gene that contains the control elements for controlling the expression of that gene. Usually the UAS contains a promoter part comprising a TATA box, and a 5'UTR. The UAS can also contain multiple cis-regulatory elements which lay in or outside the promoter or the 5'UTR.

5'UTR (5' untranslated region) generally relates to the part of a gene starting from the transcription initiation site to the translation initiation site or ATG start codon of the coding region of the gene. The 5'UTR can contain an intron which harbors control elements (such as the E2F control element of the present invention), which can then be spliced out after transcription. This removal of the control elements does not affect the specificity of (or the control on) the expression, but can contribute to the stability of the transcript. Alternatively the 5'UTR can contain an exon which harbors the control elements. Therefore the expression "transcribed region" can also mean "5' untranslated leader" and "5' transcribed, untranslated region".

It is not excluded that the E2F control element of the present invention will also work when present in the translated region of the gene, or in an intron of the gene.

In conclusion the E2F control element according to the present invention can reside in the gene in any position downstream of the transcription initiation site, which is in the transcribed region.

A "control element" as used herein means a regulatory element that regulates the expression of a gene. In case the regulatory element resides on the same DNA strand as the gene that it controls, the expression "cis-regulatory element" is used. The expression "regulatory sequence", "box" or "motif" can be used alternatively. Therefore also the terms "E2F control element", "E2F box", "E2F cis-element" or "E2F motif" are interchangeable.

"ectopic expression" means upregulation (for instance via overexpression, induction etc..) or downregulation (for instance via antisense, cosupression, etc..) of expression.

"To obtain or to drive expression of a gene" when used herein means the expression of a transgene or an endogenous gene. This expression can be again upregulated or downregulated. "To drive expression" herein thus generally means influencing the level of expression.

"Functional part" as used herein means a portion that contains all or at least one of the regulatory elements.

"Combined with", means to be coupled together so that both parts are functional at the same time and that there activity can mutually influence the activity of the other combined part.

Those skilled in the art will be aware that the invention described herein is subject to variations and modifications other than those specifically described. It is to be understood that the invention described herein includes all such variations and modifications. The invention also includes all such steps, features, compositions and compounds referred to or indicated in this specification, individually or collectively, and any and all combinations of any or more of said steps or features.

Throughout this specification, unless the context requires otherwise the word "comprise", and variations such as "comprises" and "comprising", will be understood to imply the inclusion of a stated integer or step or group of integers or steps but not the exclusion of any other integer or step or group of integers or steps.

Conventional hybridisation conditions are described e.g. (Sambrook et al, 1989) but the skilled craftsman will appreciate that numerous different hybridisation conditions can be designed in function of the known or the expected homology and/or length of the nucleic acid sequence. With specifically hybridising is meant hybridising under stringent conditions. Specific conditions for "specifically hybridizing" are for example: hybridising under stringent conditions such as a temperature of 60°C followed by washes in 2XSSC, 0.1XSDS, and 1X SSC, 0.1X SDS.

Genes and coding sequences essentially encoding the same protein but isolated from different sources can consist of substantially divergent nucleic acid sequences. Reciprocally, substantially divergent nucleic acid sequences can be designed to effect expression of essentially the same protein. Said nucleic acid sequences are the result of e.g. the existence of different alleles of a given gene, or of the degeneracy of the genetic code or of differences in codon usage. Allelic variants are further defined as to comprise single nucleotide polymorphisms (SNPs) as well as small insertion/deletion polymorphisms (INDELs; the size of INDELs is usually less than 100 bp).

An isolated nucleic acid as used herein refers to nucleotides, either ribonucleotides or deoxyribonucleotides or a combination of both, in a polymeric form of any length. Said terms furthermore include double-stranded and single-stranded DNA and RNA. Said terms also include known nucleotide modifications such as methylation, cyclization and 'caps' and substitution of one or more of the naturally occurring nucleotides with an analogue such as inosine.

DNA sequences as defined in the current invention can be interrupted by intervening sequences. With "intervening sequences" is meant any nucleic acid sequence which disrupts a coding sequence comprising said inventive DNA sequence or which disrupts the expressible format of a DNA sequence comprising said inventive DNA sequence. Removal of the intervening sequence restores said coding sequence or said expressible format. Examples of intervening sequences include introns, mobilizable DNA sequences such as transposons and DNA tags such as e.g. a T-DNA. With "mobilizable DNA sequence" is meant any DNA sequence that can be mobilised as the result of a recombination event.

Accordingly the present invention also envisage the nucleic acids and vectors as described interrupted by intervening sequences.

With "vector" or "vector sequence" is meant a DNA sequence, which can be introduced in an organism by transformation and can be stably maintained in said organism. Vector maintenance is possible in e.g. cultures of *Escherichia coli, A. tumefaciens, Saccharomyces cerevisiae* or *Schizosaccharomyces pombe*. Other vectors such as phagemids and cosmid vectors can be maintained and multiplied in bacteria and/or viruses. Vector sequences generally comprise a set of unique sites recognised by restriction enzymes, the multiple cloning site (MCS), wherein one or more non-vector sequence(s) can be inserted.

"Expression vectors" form a subset of vectors, which, by virtue of comprising the appropriate regulatory sequences, enable the creation of an expressible format for the inserted non-vector sequence(s), thus allowing expression of the protein encoded by said non-vector sequence(s). Expression vectors are known in the art enabling protein expression in organisms including bacteria (e.g. *E. coli*), fungi (e.g. *S. cerevisiae, S. pombe, Pichia pastoris*), insect cells (e.g. baculoviral expression vectors), animal cells (e.g. COS or CHO cells) and plant cells.

Reference herein to a "promoter" is to be taken in its broadest context and includes the transcriptional regulatory sequences derived from a classical eukaryotic genomic gene, including the TATA box which is required for accurate transcription initiation, with or without a CCAAT box sequence and additional regulatory elements (i.e. upstream activating sequences, enhancers and silencers) which alter gene expression in response to developmental and/or external stimuli, or in a tissue-specific manner.

The term "promoter" also includes the transcriptional regulatory sequences of a classical prokaryotic gene, in which case it may include a -35 box sequence and/or a -10 box transcriptional regulatory sequences.

The term "promoter" is also used to describe a synthetic or fusion molecule or derivative, which confers, activates or enhances expression of a nucleic acid molecule in a cell, tissue or organ.

Promoters may contain additional copies of one or more specific regulatory elements, to further enhance expression and/or to alter the spatial expression and/or temporal expression of a nucleic acid molecule to which it is operably connected. Such regulatory elements may be placed adjacent to a heterologous promoter sequence to drive expression of a nucleic acid molecule in response to e.g. copper, glucocorticoids, dexamethasone, tetracycline, gibberellin, cAMP, abscisic acid, auxin, wounding, ethylene, jasmonate or salicylic acid or to confer expression of a nucleic acid molecule to specific cells, tissues or organs such as meristems, leaves, roots, embryo, flowers, seeds or fruits.

In the context of the present invention, the promoter preferably is a plant-expressible promoter sequence. Promoters, however, that also function or solely function in non-plant cells such as bacteria, yeast cells, insect cells and animal cells are not excluded from the invention.

By " plant development and/or plant morphology and/or biochemistry and/or physiology" is meant that one or more developmental and/or morphological and/or biochemical and/or physiological characteristics of a plant is altered by the performance of one or more steps pertaining to the invention described herein.

"Plant development" or the term "plant developmental characteristic" or similar term shall, when used herein, be taken to mean any cellular process of a plant that is involved in determining the developmental fate of a plant cell, in particular the specific tissue or organ type into which a progenitor cell will develop. Cellular processes relevant to plant development will be known to those skilled in the art. Such processes include, for example, morphogenesis, photomorphogenesis, shoot development, root development, vegetative development, reproductive development, stem elongation, flowering, and regulatory mechanisms involved in determining cell fate, in particular a process or regulatory process involving the cell cycle.

"Plant morphology" or the term "plant morphological characteristic" or similar term will, when used herein, be understood by those skilled in the art to refer to the external appearance of a plant, including any one or more structural features or combination of structural features thereof. Such structural features include the shape, size, number, position, colour, texture, arrangement, and patternation of any cell, tissue or organ or groups of cells, tissues or organs of a plant, including the root, stem, leaf, shoot, petiole, trichome, flower, petal, stigma, style, stamen, pollen, ovule, seed, embryo, endosperm, seed coat, aleurone, fibre, fruit, cambium, wood, heartwood, parenchyma, aerenchyma, sieve element, phloem or vascular tissue, amongst others.

"Plant biochemistry" or the term "plant biochemical characteristic" or similar term will, when used herein, be understood by those skilled in the art to refer to the metabolic and catalytic processes of a plant, including primary and secondary metabolism and the products thereof, including any small molecules, macromolecules or chemical compounds, such as but not limited to starches, sugars, proteins, peptides, enzymes, hormones, growth factors, nucleic acid molecules, celluloses, hemicelluloses, calloses, lectins, fibres, pigments such as anthocyanins, vitamins, minerals, micronutrients, or macronutrients, that are produced by plants.

"Plant physiology" or the term "plant physiological characteristic" or similar term will, when used herein, be understood to refer to the functional processes of a plant, including developmental processes such as growth, expansion and differentiation, sexual development, sexual reproduction, seed set, seed development, grain filling, asexual reproduction, cell division, dormancy, germination, light adaptation, photosynthesis, leaf expansion, fiber production, secondary growth or wood production, amongst others; responses of a plant to externally-applied factors such as metals, chemicals, hormones, growth factors, environment and environmental stress factors (eg. anoxia, hypoxia, high temperature, low temperature, dehydration, light, day length, flooding, salt, heavy metals, amongst others), including adaptive responses of plants to said externally-applied factors.

The term "cell cycle" means the cyclic biochemical and structural events associated with growth and with division of cells, and in particular with the regulation of the replication of DNA and mitosis. Cell cycle includes phases called: G0, Gap1 (G1), DNA synthesis (S), Gap2 (G2), and mitosis (M). Normally these four phases occur sequentially, however, the cell cycle also includes modified cycles wherein one or more phases are absent resulting in modified cell cycle such as endomitosis, acytokinesis, polyploidy, polyteny, and endoreduplication.

"Plant cell" comprises any cell derived from any plant and existing in culture as a single cell, a group of cells or a callus. A plant cell may also be any cell in a developing or mature plant in culture or growing in nature.

"Plant" or "Plants" comprise all plant species which belong to the superfamily Viridiplantae. The present invention is applicable to any plant, in particular a monocotyledonous plants and dicotyledonous plants including a fodder or forage legume, ornamental plant, food crop, tree, or shrub selected from the list comprising *Acacia spp., Acer spp., Actinidia spp.,Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chaenomeles spp.,Cinnamomum cassia, Coffea arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Diheteropogon amplectens, Dioclea spp, Dolichos spp., Dorycnium rectum, Echinochloa pyramidalis, Ehrartia spp., Eleusine coracana, Eragrestis spp., Erythrina spp., Eucalyptus spp., Euclea schimperi, Eulalia villosa, Fagopyrum spp., Feijoa sellowiana, Fragaria spp., Flemingia spp, Freycinetia banksii, Geranium thunbergii, Ginkgo biloba, Glycine javanica, Gliricidia spp, Gossypium hirsutum, Grevillea spp., Guibourtia coleosperma, Hedysarum spp., Hemarthia altissima, Heteropogon contortus, Hordeum vulgare, Hyparrhenia rufa, Hypericum erectum, Hyperthelia dissoluta, Indigo incarnate, Iris spp., Leptarrhena pyrolifolia, Lespediza spp., Lettuca spp., Leucaena leucocephala, Loudetia simplex, Lotonus bainesii, Lotus spp., Macrotyloma axillare, Malus spp., Manihot esculenta, Medicago sativa, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativum, Podocarpus totara, Pogonarthria fleckii, Pogonarthria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes* *grossularia, Ribes spp., Robinia pseudoacacia, Rosa spp., Rubus spp., Salix spp., Schyzachyrium sanguineum, Sciadopitys verticillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp.Vitis vinifera, Watsonia pyramidata, Zantedeschia aethiopica, Zea mays,* amaranth, artichoke, asparagus, broccoli, brussel sprout, cabbage, canola, carrot, cauliflower, celery, collard greens, flax, kale, lentil, oilseed rape, okra, onion, potato, rice, soybean, straw, sugarbeet, sugar cane, sunflower, tomato, squash, and tea, amongst others, or the seeds of any plant specifically named above or a tissue, cell or organ culture of any of the above species.

### Description of Figures

### Figure 1. Cell cycle-regulated expression of RNR1b gene.

**(A)** Tobacco BY2 cells were synchronized by aphidicolin and cell cycle progression was monitored by measurements of DNA synthesis (filled circles) and mitotic index (triangles). **(B)** RNA samples (20 µg) prepared from cells taken every hour were blotted and successively hybridized to the coding region of BY2RNR1a cDNA and to the 3' specific region of the RNR1b cDNA. The loading control **(C)** was the EF-1 alpha gene, shown to be constitutively expressed during the cell cycle progression (Regad et al., 1995).

### Figure 2. Kinetics of RNR1b transcript accumulation in synchronized tobacco cells following a replication block in mid-S phase.

**(A)** Cells synchronized with aphidicolin were treated with HU (60mM) in the middle of the first S phase (arrowhead). Samples were collected every hour after treatment and processed for RNA analysis. DNA synthesis was measured by ³HTTP pulse labeling in the control (filled circles, CDNA) or HU treated cells (open circles, HUDNA). 20 µg of RNA from the control (filled triangles) or the HU treated cells (open triangles) were blotted and successively hybridized to the RNR1 coding region (R1). The plotted values are relative to the maximal level of RNA obtained in the control.

**(B)** 20 µg of RNA from the control (filled triangles) or the HU treated cells (open triangles) were blotted and successively hybridized to the 3' specific probe of the RNR1b cDNA (R1B). The equal loading of the gel was confirmed by probing the blot with the EF-1 alpha gene. The plotted values are relative to the maximal level obtained in the control.

### Figure 3. Nucleotide Sequence of the Tobacco RNR1b UAS.

Putative cis-elements are indicated by arrows and the putative TATA box is framed.

### Figure 4. Involvement of E2F factor in nuclear complex bound to RNR1b UAS-E2F element.

Wild type (WT), E2F-mutated (MU) or unrelated (U) double stranded oligonucleotides were used in the gel shift assays. **(A)** Gel shift was performed in presence of nuclear extracts from mid-log phase BY2 cells with the ³²P-labelled WT probe; free probe (PR); complexes with 6 µg of nuclear extract (EX); competitions with a 50 to 200-fold molar excess of the unlabelled WT probe, of the E2F-mutated oligonucleotides (MU), and of an unrelated oligonucleotide (U). **(B)** Competition assay with the antibody directed against the DNA binding domain of human E2F5 (alpha E2F5, 2 and 4µl) and an unrelated antibody directed against alpha-tubulin. **(C)** Binding of a purified tobacco E2F factor (EX, 300 ng of E2F protein). In competition assays, 200 fold excess of the wild type (WT) and E2F-mutated (MU) oligonucleotides were used as well as 2µl of antibody directed against an unrelated antibody (C) or the DNA binding domain of human E2F5 factor (alpha-E2F5). Specific E2F complexes are indicated by arrows.

### Figure 5. E2F element of the RNR1b UAS drives specific G1/S induction and s phase activity.

RNR1b UAS constructs were fused to the luciferase reporter gene: the wild type UAS (WT) or E2F-mutated UAS (MU). LUC activities were measured in transgenic BY2 cell lines containing the UAS constructs, as described in example 5. **(A)** Tobacco BY2 cells were synchronized by aphidicolin and cell cycle progression was monitored by measurements of DNA synthesis (filled circles) and mitotic index (filled triangles). **(B)** LUC activities of cells harvested at different time-points of the cell cycle. RLU: relative light unit. Results were reproducible in three individual experiments.

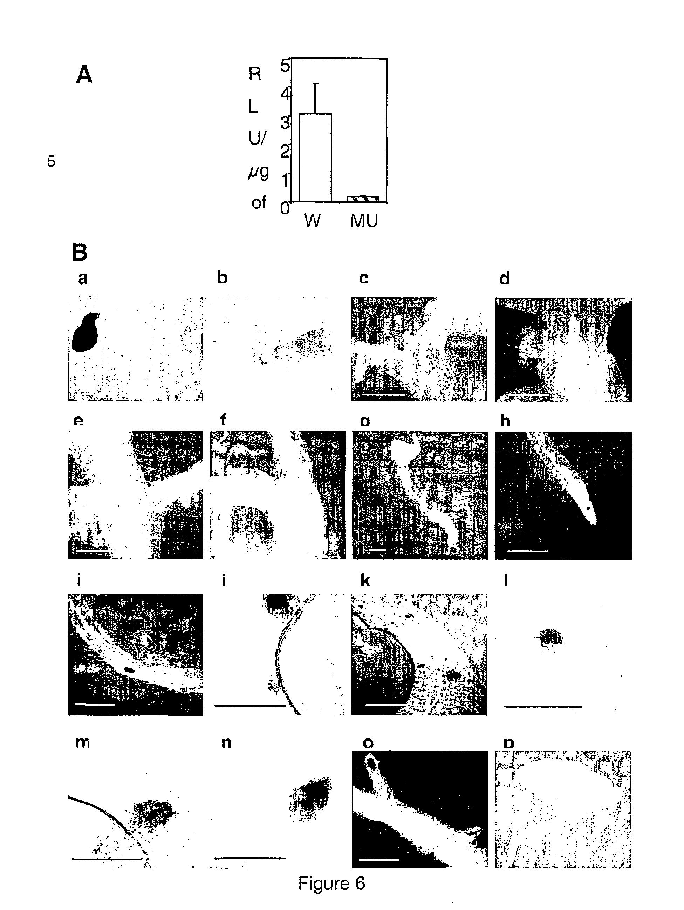

### Figure 6. E2F element confers meristematic activity to the RNR1b UAS

WT or E2F-mutated UAS were fused to the GUS reporter gene. (A) GUS activities were measured in young plantlets (10 samples for each transgenic plant) issued from the F1 generation. (B) Histological analysis of the GUS staining was performed on plantlets carrying either the WT construct (a to o) or the E2F-mutated construct (p): young seedling (a), older seedling (b), young leaves (c-d), axillary buds (e-f), root tips (g-h), secondary roots (i to o). Bars represent 0.5 mm.

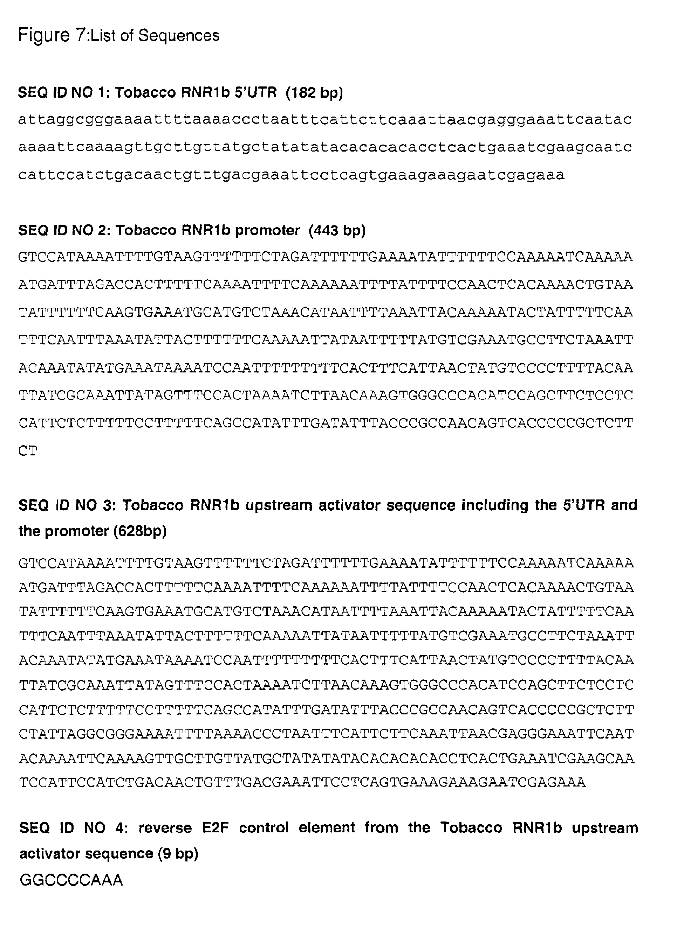

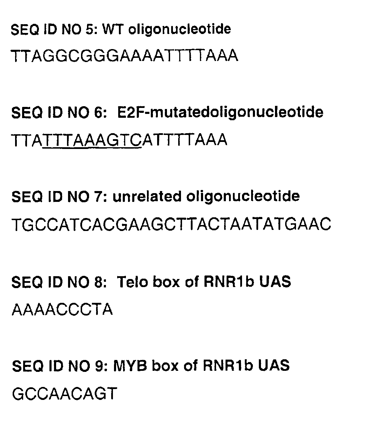

**Figure 7.** List of sequences

### Examples

### Example 1

### Plant cell culture and synchronization

The tobacco BY2 cell suspension was maintained by weekly subculture as described (Nagata et al, 1992). For synchronization freshly subcultured stationary phase cells were treated with aphidicolin (3 µg/ml, Sigma Chemicals) for 24 hours and extensively washed. DNA synthesis and mitotic index were monitored as described (Reichheld et al., 1995).

### Cell cycle expression of the RNR1b gene is restricted to G1/S transition phase and to the s phase

The level of RNR1b transcripts was measured throughout the cell cycle in synchronized BY2 tobacco cells (Fig. 1). Suspension-cultured cells were synchronized by aphidicholin as described (Nagata et al, 1992). After removal of the drug, cells arrested in S phase progressed synchronously through the cell cycle, reaching a maximal mitotic index at 8 hr (Fig. 1*A*). DNA synthesis and RNR mRNA levels were monitored for 15 hours (Fig. 1*B*). Tobacco RNR1 genes are cell cycle regulated and show maximum expression in S phase and a significant gene induction at the G2/M transition. Probing with the coding region of tobacco RNR1a cDNA, both RNR1 genes (RNR1a and RNR1b) were detected since their coding region share 89.2 % sequence identity (Chabouté et al, 1998). However, using a 3' specific probe, it is shown that RNR1b expression is strictly restricted to S phase. Therefore, the G2/M expression level detected with the coding region probe could be due to induction of the RNR1 a gene.

### Example 2

### Modulation of RNR1b gene expression in response to replication block

To determine the response of the RNR1b gene to a block in DNA synthesis hydroxyurea (HU) was used. HU directly blocks RNR activity by specifically quenching the tyrosyl radical of the small subunit (Krakoff et al, 1968). The cells are deprived of newly synthesized dNTPs and DNA replication is blocked. HU applied to mid-S phase cycling cells led to rapid decrease in DNA synthesis compared to the control (Fig. 2A, curves CDNA and HUDNA). Immediately after HU treatment, the level of both RNR1 transcripts (RNR1a and RNR1b mRNA) and the RNR1b mRNA levels decreased parallel to the control. This is shown in Figure 2A, (curves HUR1 and CR1) and in Figure 2B curves HUR1B and CR1B respectively. One hour later, i.e. approximately two hours after drug treatment, total RNR1 transcript level increased sharply to a maximal level 10 times higher than the control (Fig. 2A curves CR1 and HUR1) while the RNR1b mRNA level was stabilized at a constant level (Fig. 2B curves HUR1B and CR1B). It therefore appears that after blocking DNA replication, RNR1a gene is strongly up-regulated compared to RNR1b gene who's mRNA level remains constant. Stabilization of the transcript level could be due either to a higher stability of the transcripts or to a prolonged activity of the RNR1b UAS. Since the level of RNR1b transcripts fluctuates in a different way than the total RNR1 mRNA, the experiments show that DNA synthesis regulates the transcription of the two RNR1 genes by different mechanisms.

### Example 3

### Sequence analysis of the tobacco RNR1b UAS

Since RNR1b gene was specifically induced during G1/S phase, the inventors further investigated the transcriptional regulation of this gene. The 628 DNA bp-upstream activator region was amplified and cloned by inverse PCR on tobacco genomic DNA using primers adjacent to the ATG start in the cDNA (accession number Y10862), as described previously (Chabouté et al, 2000). Sequence analysis revealed several potential cis-elements (Fig. 3), notably a reverse E2F-binding site at - 177bp (GCGGGAAA) from the ATG start. In the vicinity of the E2F element, one putative MYB binding site (CAACAG) as well as one Telobox (AAACCCTAA) were found at - 204 and -164 bp, respectively, from the start codon. Upstream from these cis-elements, a putative TATA box was found at - 405 bp. Three types of MYB target sequences have been identified in plants, one of which (YAACNG) (Romero et al, 1998), is similar to those found in animals (Faisst et al, 1992). The MYB binding site in the RNR1b promoter shows strong homology to this sequence. Teloboxes, internal telomeric repeats, have been identified in plant promoters from genes encoding PCNA or ribosomal proteins that are expressed in cycling cells (Manevski et al, 2000).

### Example 4

### Nuclear extracts and electrophoretic mobility shift assays

Tobacco BY2 cells were harvested by centrifugation at 200 g for 10 min and frozen in liquid nitrogen. Pelleted cells were resuspended in 4 ml/g of buffer A containing 25 mM Tris-HCI pH 6.5, 0.45 M sucrose, 5mM MgCl₂, 5mM beta-mercaptoethanol, 0.5 mM PMSF (phenylmethylsulphonyl fluoride, Sigma), 250 µg/l pepstatin (Sigma), 500 µg/l leupeptin (Sigma) and were broken with a French press apparatus (3000 psi). Nuclear extracts were prepared as described (Shen & Gigot, 1997) and stored in aliquots at -80°C. The double stranded oligonucleotides used in gel shifts experiments were:
wild type (WT): TTAGGCGGGAAAATTTTAAA (SEQ ID NO 5),
E2F-mutated (MU): TTATTTAAAGTCATTTTAAA (SEQ ID NO 6), and
an unrelated oligonucleotide: TGCCATCACGAAGCTTACTAATATGAAC(SEQ ID NO 7). Assays were done as reported (Chabouté et al, 2000). Two antibodies were used for competition assays: anti-E2F5 (Santa Cruz Biotechnology, Santa Cruz, CA) directed against the DNA binding domain of human E2F5 and anti alpha-tubulin (Amersham Pharmacia Biotech).

### E2F transcription factor is part of the nuclear complex associated with the RNR1b E2F- element

In animals, E2F control elements are crucial for the G1/S induction of cell cycle regulated genes, which usually are regulated by more than one E2F site. The role of the E2F site in the RNR1b UAS was investigated. First, the capacity of the E2F element to bind nuclear complexes was invetsigated. Gel retardation assays were performed with nuclear extracts prepared from BY2 exponentially growing cells and ³²P-labelled double-stranded oligonucleotides carrying either the E2F control element (WT) or its mutated version (MU). In presence of nuclear extract (EX), two retarded complexes (I and II) were detected with the E2F probe and titrated out by an excess of unlabeled wild-type E2F oligonucleotide (Fig. 4A, WT). Neither the oligonucleotide mutated in the E2F site (MU) nor an unrelated oligonucleotide (U) were able to displace the complex (I), indicating that this complex has a specific binding activity for the E2F control element. To show that an E2F transcription factor is part of the complex (I), antibody competition was used in the bandshift experiment. Since the DNA binding domain of E2F factors is conserved in tobacco and mammals (75 % amino acid identity) (Sekine et al, 1999), an antibody raised against the DNA binding domain of the human E2F5 factor was used. This antibody was previously shown to specifically recognize tobacco E2F factor (Chabouté et al, 2000). Increasing the concentration of E2F5 antibody (2 to 4 µl) (Fig. 4*B*, αE2F5), dramatically decreases the E2Fcontrol element-binding activity of the complex (I) compared to the control (C) using an unrelated antibody. In addition, a tobacco E2F factor strongly binds the E2F probe (Fig. 4C). Competition with an excess of unlabelled probe (WT) but not by the E2F-mutated oligonucleotide (MU), reduced the signal significantly, showing that the interaction of the tobacco E2F factor with the RNR1B E2F element is specific. Moreover, the E2F5 antibody prevents binding of the E2F factor to the wild type probe whereas an unrelated antibody (C) has no effect. It therefore appears that purified E2F factor or an E2F-containing nuclear complex specifically binds to the E2F control element of the RNR1b UAS.

### Example 5

### UAS-luciferase reporter gene constructs

The CaMV 35S promoter was removed from the luciferase-intron reporter gene plasmid pLuk07 (Mankin et al, 1997) by a KpnI-NcoI digestion. The WT and E2F-mutated UAS were amplified using specific primers carrying Kpnl and NcoI sites respectively. The amplified products were sequenced and subcloned in the pLuk07 plasmid. The Kpnl-Xbal fragments carrying the fusion RNR1b UAS-luciferase were subcloned in the KpnI-XbaI sites of the binary vector pCGN1549 (Calgene).

Plasmids constructs were introduced into *Agrobacterium tumefaciens* LBA4404 and used to transform tobacco BY2 cells, as described (An et al, 1987). About 500 to 1000 kanamycin-resistant calli were pooled and grown as suspension cultures. The transgenic cell suspensions were maintained by subculturing 2 ml of stationary phase cells in 80 ml of fresh medium supplemented with carbenicillin (500 µg/ml) and kanamycin (100 µg/ml). After 4 rounds of subculture, carbenicillin was omitted from the medium.

### Luciferase assay

2 ml of cells were washed twice in PBS buffer (140 mM NaCI, 2.7 mM KCI, 1.5 mM K₂H₂PO₄, Na₂HPO₄ pH 7.4) and lysed by a 10 min incubation at room temperature in 200 µl of lysis buffer (100 mM potassium phosphate buffer pH 7.8, 1mM DTT, and 0.2 % Triton X-100). After centrifugation at 6000 rpm for 3 min, the supernatant was frozen in liquid nitrogen and stored at -80°C. Luciferase activity was measured using the Luciferase assay kit (Tropix) in a microplate luminometer (TR 717 Tropix Perkin Elmer, Applied Biosystems) according to the manufacturer's instructions.

### The E2F site is sufficient to drive G1/S phase induction and S phase specific activity of the RNR1b UAS

To investigate the role of the E2F site in the activity of the RNR1b UAS, wild type or the E2F-mutated UAS (using the same nucleotide changes as in gel shift assays) were fused to the luciferase reporter gene. BY2 mid-log phase cells were stably transformed by the different constructs in Agrobacterium. Pools of 1000 individual clones were cultured as cell suspensions and analyzed. After synchronization of the transgenic lines by aphidicholin, DNA synthesis and the mitotic index were monitored throughout the cell cycle progression (Fig. 5A). In both transgenic lines (WT and MU), a maximal mitotic index of 40% was reached at 9 hr and DNA synthesis was maximal at 19 hr. Luciferase activity was monitored in parallel. The activity of the WT construct was low in G2 (4-6 hr), M(6-14 hr) and G1 (14-18 hr) phases and increased concurrently with DNA synthesis in S phase (Fig. 5*B*). In contrast, the luciferase (LUC) activity of the mutated construct remained very low throughout S phase (19-21hr). The inventors conclude that the E2F control element is sufficient for both G1/S induction and the maximal S phase activity of the RNR1 UAS in synchronized tobacco cells.

### Example 6

### UAS-GUS reporter gene constructs

The WT UAS was subcloned in the Xbal site of the pBluescript (pKS) vector and the E2F site was mutated by PCR-based site-directed mutagenesis. Each construct was fused to the GUS reporter gene in the Xbal-Smal site of the binary vector pBI 101. Plasmid constructs were introduced into *A. tumefaciens* strain LBA 4404. Tobacco plants (*Nicotiana tabacum* L. cv SR1) were transformed by the leaf-disc method (Horsch et al, 1985). Plants were grown *in vitro* and in the greenhouse. Seeds were collected from 10 independent primary regenerants and germinated on kanamycin for analysis of the F1 progeny.

### GUS assays

GUS histochemical staining was carried out according to Jefferson et al. (Jefferson et al, 1987) on F1 plants issued from independent transformation events. 8 to 10 plantlets from each line were tested at the germination stage. Hand-cut sections were viewed by bright-or dark field microscopy.

Quantitative GUS assays were carried out using the GUS light kit (Tropix). Frozen plantlets were grounded in presence of 200 µl of lysis buffer (50 mM phosphate sodium buffer pH 7, 10 mM EDTA, 0.1 % Triton X100, 10mM βSH). After centrifugation at 10000 rpm for 2 min, the supernatant was stored at 4°C. GUS activity was measured in the same luminometer as described above according to the manufacturer's instructions.

### The E2F site drives meristematic activity of the RNR1b UAS

To assay the tissue specificity of the RNR1b UAS and the role of the E2F control element, both the wild type and the E2F-mutated UAS were fused to the GUS reporter gene. SR1 tobacco leaf discs were stably transformed by the constructs cloned in Agrobacterium. After regeneration of primary transformants, luminometric quantification of GUS activity was performed on F1 plants. Young plantlets carrying the wild type construct showed high GUS activity while almost no activity was found in plants with the mutated UAS (Fig. 6A). To determine whether the UAS drives tissue-specific expression, histological analysis of GUS expression was done. The wild type UAS was able to drive GUS expression in meristematic tissues of developing seedlings (Fig. 5*B*, a-o). Strong GUS staining was observed in cotyledons of young seedlings (a) but as the plants grew, staining in cotyledons became weak and speckled (b). Significant staining was also observed in young leaves (c) but not in the apical meristem (d). Primary root tips (g) were labelled but the signal decreased as plantlets grew (h). Higher GUS activity was detected in shoot apex of axillary buds and in leaf primordia (e-f) or in the meristematic zone of lateral root primordia : first in dividing cells in the pericycle (i-j), then, in lateral-root primordia before root formation (k-l), and finally, in emerging root tip (m to o). In contrast, no GUS activity was found in plants carrying the mutated E2F UAS-GUS fusion (p). Consequently, it appears that wild type RNR1b UAS activity is primarily restricted to meristematic tissues in tobacco plantlets and the E2F control element is required for this activity.

### Example 7

In a particular application of the present invention, the RNR UAS is operably linked to a plant E2F gene and this expression cassette is further cloned in a suitable vector and subsequently introduced into a plant. In one example, the SEQ ID NO 3 is genetically fused to the coding sequence of the Tobacco E2F gene, cloned into a binary vector and transformed to Arabidopsis and Rice using standard transformation techniques. Arabidopsis plants are transformed with the flower dip method and agrobacterium-mediated rice transformation is performed on embryogenic, scutellum-derived callus. Alternatively the rice or Arabidopsis or other plant E2F gene is used and/or other plants are transformed. In this way the E2F gene is specifically overexpressed in meristematic cells and is not mis-expressed in other plant tissues. Moreover, the expression level in the meristem is additionally increased because of the auto feedback loop generated by the expression of the E2F gene and the E2F regulation of the UAS.

### Example 8

In a particular application, the UAS comprising the E2F control element of the invention is used to drive the expression of a plant E2F gene and a plant DP gene in the same cell. This is achieved by linking the coding regions of the E2F gene and the DP gene each to one site of a bi-directional UAS as described in example 10. Alternatively, simultaneous expression of the E2F gene and the DP gene is achieved by using two separate UAS comprising the E2F control element of the present invention or by constructing a dicistronic operon driven by one UAS, comprising the E2F control element of the present invention. These expression cassettes are than cloned in a binary vector and transformed to a plant, using standard methods. The plants transformed with the E2F and the DP gene under the control of the UAS comprising the E2F control element of the invention, have an increased expression level of these genes in their meristem and/or in the G1/S transition and S phase of the cell cycle. Via an auto feedback loop, the produced E2F and DP proteins further increase their own expression level via control of the E2F element. This effect can be very strong.

### Example 9

By the use of the E2F control element of the present invention, preferably in the context of the 5'UTR of RNR1b, any promoter can be made active in meristem and/or in the G1/S transition and the S phase: this means that the activity of a plant promoter can be induced in the meristem and/or in the G1/S transition and/or in the S phase of the cell cycle, by linking the promoter sequence to the E2F control element of the present invention. In this way a hybrid expression cassette can be designed according to the need. In a particular application of the present invention the 35S promoter, which is known to be a strong promoter but which is not active in meristem, is fused in front of SEQ ID NO 1. In this way the strong promoter becomes active in the G1/S transition and S phase and/or in the meristem.

In the scope of the present invention is to couple the E2F element of the present invention in the context of a 5'UTR, e.g. the 5'UTR of RNR1, e.g. SEQ ID NO 1, to a promoter to obtain an expression cassette which is active in the G1/S transition and S phase and/or in meristematic cells.

Alternatively, in the scope of the present invention is to couple the E2F element of the present invention in the context of a 5'UTR, e.g. the 5'UTR of RNR1, e.g. SEQ ID NO 1, to a gene to obtain G1/S induction and S phase expression and/or meristematic expression regardless of the promoter that is coupled to that gene.

The control of expression as described above can be further optimized by incorporating also other cis-regulatory elements of the RNR1b UAS, such as the Myb box and the Telobox, in the hybrid expression cassette or in the hybrid gene.

In a related application the E2F element of the present invention is incorporated in an UAS in multiple copies in order to improve the activity of the UAS and/or to obtain a stronger regulation of an existing UAS. In the RNR1b UAS an extra copy E2F of the present invention is introduced to obtain a stronger expression of the regulated gene. In another example, the E2F control element of the present invention is incorporated in the UAS of G1/S or S phase specific genes (regardless of the presence of a native E2F control element as in the case of cdc6) to obtain a better regulation and/or a stronger expression. The control of expression can be further optimized by incorporating also other cis-regulatory elements of the RNR1b UAS, such as the Myb box and the Telobox, in the hybrid UAS.

### Example 10

Construction of a bi-directional UAS, which has meristem and G1/S transition and S phase specificity and that can drive two genes at the same time, can be achieved by coupling the functional parts of UAS of the present invention (including the promoter region with a TATA sequence and the 5'UTR comprising the E2F control element of the present invention) behind another TATA-box-comprising promoter element which is itself preceded by a 5'UTR comprising the E2F control element of the present invention. The control of expression can be further optimized by incorporating also other cis-regulatory elements of the RNR1b UAS, such as the Myb box and the Telobox, in the bi-directional UAS.

### References

Afroze, B. & Husain, M. (2000) J. Biol. Chem. 275, 9062-9069.
An, G. (1987) Methods Enzymol. 153, 292-305.
Bartley, P. A., Lutwyche, J. K. & Gonda, T. J. (2001) Blood Cells Mol. Dis. 27, 409-415.
Chabouté et al, 1998. Plant Mol. Biol. 38, 797-806
Chabouté et al, 2000. Plant Cell 12: 1987-2000
Chabouté, M. E., Clément, B., Sekine, M., Philipps, G. & Chaubet-Gigot, N. (2000).
Chabouté, M. E., Combettes, B., Clément, B., Gigot, C. & Philipps, G. (1998) Plant Mol. Biol. 38, 797-806.
Chaubet, N., Flénet, M., Clément, B., Brignon, P. & Gigot, C. (1996) Plant J. 10, 425-435.
De Jager, S. M., Menges, M., Bauer, U. M. & Murray, J. A. H. (2001) *Plant Mol. Biol*. 47, 555-568.
Durfee, T., Feiler, H. S. & Gruissem, W. (2000) *Plant Mol. Biol.* 43, 635-642.
Egelkrout, E. M., Robertson, D. & Hanley-Bowdoin, L. (2001) *Plant Cell 13, 1437*-1452.
Faisst, S. & Meyer, S. (1992) Nucleic Acids Res. 20, 3-26.
Horsch, R. B., Fry, J. E., Hoffmann, N. L., Eichholtz, D., Rogers, S. G. & Fraley, R. T. (1985) Science 227, 1229-1231.
Ito, M. (1998) in Plant cell division., eds. Francis, D., Dudits, D. & Inzé, D. (Portland Press Ldt., London), pp. 165-186.
Jefferson, R. A., Kavanagh, T. A. & Bevan, M. W. (1987) EMBO J. 6, 3901-3907.
Kosugi et al, 1997. Plant Cell 9: 1607-1619
Kosugi, S., Suzuka, I. & Ohashi, Y. (1995) Plant J. 7, 877-886
Kosugi, S. & Ohashi, Y. (2002) Plant J. 29(1), 45-56
Krakoff, I. H., Brown, N. C. & Reichard, P. (1968) Cancer Res. 28, 1559-1565.
Magyar Z, Atanassova A, De Veylder L, Rombauts S, Inze D (2000) FEBS Lett. 486(1):79-87.
Mankin, S. L., Allen, G. C. & Thompson, W. F. (1997) Plant Mol. Biol. Rep. 15, 186-196
Nagata, T., Nemoto, Y. & Hazezawa, S. (1992) Int. Rev. Cyt. 132, 1-30.
RamirezParra, E., Xie, Q., Boniotti, M. B. & Gutierrez, C. (1999) *Nucleic Acids Res.* 27, 3527-3533.
Regad et al. 1995 Mol Gen Genet 248, 703-11
Reichard, P. (1988) *Ann. Rev. Biochem.* 57, 349-374
Reichheld, J.P., Sonobe, S., Clément, B., Chaubet, N. & Gigot, C. (1995) *Plant J*. 7, 245-252.
Romero, I., Fuertes, A., Benito, M. J., Malpica, J. M., Leyva, A. & Paz-Ares, J. (1998) Plant J. 14, 273-284.
Sambrook, J., Fritsch, E. F., and Maniatis, T. (1989). "Molecular Cloning: A Laboratory Manual." Cold Spring Harbor Laboratory Press.
Sekine, M., Ito, M., Uemukai, K., Maeda, Y., Nakagami, H. & Shinmyo, A. (1999) *FEBS Lett.* 460, 117-122.
Shen, W.H. & Gigot, C. (1997) *Plant Mol. Biol*. 33, 367-379.
Taoka, K.-I., Kaya, H., Nakayama, T., Araki, T., Meshi, T. & Iwabuchi, M. (1999) Plant J. 18, 611-623.

## Claims

1. Use of an E2F control element to obtain G1/S-phase and/or S-phase and/or meristem-specific gene expression in plants, **characterized in that** said E2F control element is present in a transcribed region of a gene of interest.

2. Use according to claim 1, wherein said E2F control element is present in a 5' UTR of a gene of interest.

3. Use according to claim 1 or 2, wherein said E2F control element is combined with a Telobox sequence.

4. Use according to any of claims 1 to 3, wherein said E2F control element is present in a 5' UTR of a plant RNR1b gene

5. Use according to any of claims 1 to 4, wherein said E2F control element is comprised within the sequence as given in SEQ ID NO 1.

6. Use of an E2F control element according to any of claims 1 to 5, wherein said transcribed region of said gene of interest is preceded by a promoter

7. Use according to any of claims 1 to 6, wherein said transcribed region is combined with a MYB box

8. Use according to claim 6, wherein said promoter is the promoter of a plant RNR1b gene

9. Use according to any of claims 1 to 8, wherein said transcribed region of said gene of interest is preceded by a sequence as given in SEQ ID NO 2

10. An isolated nucleic acid, comprising an upstream activator sequence comprising:
(a) at least one promoter sequence or a functional part thereof, and
(b) at least one 5' UTR or a functional part thereof comprising at least one E2F control element.

11. An isolated nucleic acid according to claim 10 comprising a sequence selected from the group consisting of :
(a) a nucleic acid comprising the DNA sequence as given in SEQ ID NO , 2 or 3 , or the complement thereof,
(b) a nucleic acid specifically hybridizing with the nucleic acid as defined in (a)
(c) a nucleic acid which is diverging, due to the differences between alleles, to a nucleotide sequence as defined in (a) or (b), and
(d) a nucleic acid consisting of the sequence as given in any of SEQ ID NOs 1, 2 or 3.

12. An isolated nucleic acid comprising in the 5' to 3' direction in the following order at least one TATA box, at least one MYB box, at least one E2F control element in the sense or reverse orientation, and a Telobox.

13. An isolated nucleic acid according to claim 12, wherein the TATA box, the MYB box, the E2F element and the Telobox are from the RNR1b genomic gene.

14. An isolated nucleic acid according toany of claims 1 to 13 comprising at least one TATA box, at least one sequence as given in SEQ ID NO 9, at least one sequence as given in SEQ ID NO 4 and at least one sequence as given in SEQ ID NO 8

15. An isolated nucleic acid according to any of claims 10 to 14 further comprising at least one hormone responsive element such as an auxin responsive element.

16. An isolated nucleic acid according to any of claims 10 to 15, wherein said nucleic acid is cDNA or genomic DNA.

17. A vector construct comprising an isolated nucleic acid according to any of claims 10 to 16

18. A vector according to claim 17 which is an expression vector wherein said isolated nucleic acid is operably inserted upstream of a gene of interest.

19. A host cell comprising an isolated nucleic acid or a vector construct according to any of claims 10 to 18.

20. A host cell of claim 19 which is a bacterial, insect, fungal, yeast, plant, animal or mammalian cell.

21. Use of a nucleic acid or vector construct according to any of claims 10 to 18 for G1/S phase specific induction and/or S phase specific expression and/ or meristem specific expression of a gene of interest

22. Use of a nucleic acid or vector construct according to any of claims 10 to 18 for monitoring the expression of a gene in the G1/S transition phase, in the S phase or in the meristem, or for monitoring the endoreduplication state of the cell.

23. Use of a nucleic acid or vector construct according to any of claims 10 to 18 to drive expression of an E2F gene.

24. Use of a nucleic acid or vector construct according to any of claims 10 to 18 to drive the expression of an E2F gene and of a DP gene.

25. Use of a nucleic acid or vector construct according to any of claims 10 to 18 to drive expression of hormone induced gene, such as an auxin induced gene or a growth regulatory gene.

26. Use of a nucleic acid or vector construct according to any of claims 10 to 18 to drive expression of a gene of interest in combination with the expression of a hormone induced or hormone responsive gene, such as an auxin induced or auxin responsive gene or a growth regulatory gene.

27. Use of a nucleic acid or vector construct according to any of claims 10 to 18 to drive expression of a gene of interest.

28. Use of an E2F control element located in a 5' UTR or a transcribed region of a gene as described in claims 1 to 9, to construct an expression cassette that is active in the G1/S transition phase and/or in the S phase and/or in meristematic cells.

29. Use of an E2F control element in a 5' UTR or a transcribed region of a gene, as described in claims 1 to 9, to construct a hybrid gene that is expressed in the G1/S transition phase and/or the S phase and/or in meristematic cells.

30. Use of an E2F control element in a 5'UTR or a transcribed region of a gene as described in any of claims 1 to 9 in multiple copies to obtain improved expression and/or improved regulation of the gene of interest.

31. Use of an E2F control element in a 5'UTR or a transcribed region of a gene as described in any of claims 1 to 9 to construct a bi-directional promoter that is active in the G1/S transition phase and/or in the S phase and/or in meristematic cells .

32. Use of an E2F control element in a 5'UTR or a transcribed region of a gene as described in any of claims 1 to 9 in combination with a hormone responsive element, such as an auxine responsive element.

33. Method for the production of transgenic plants comprising introducing into a plant cell, a nucleic acid or a vector construct according to any of claims 10 to 15.

34. Method for ectopic expression of an E2F gene in a plant cell comprising:
(a) operably linking the upstream activator sequence of an RNR1 b gene to an E2F gene in a nucleic acid construct, and
(b) introducing said nucleic acid construct into a plant cell.

35. Method for ectopic expression of a E2F gene and a DP gene in a plant cell comprising:
(a) operably linking the upstream activator sequence of an RNR1b gene to an E2F gene and/or to a DP gene in (a) nucleic acid construct(s), and
(b) introducing said nucleic acid constructs into a plant cell.

36. Method for ectopic expression of a hormone regulated gene, such as an auxin regulated gene, in a plant cell comprising:
(a) operably linking the upstream activator sequence of an RNR1b gene to a hormone regulated gene, such as an auxin regulated gene in a nucleic acid construct, and
(b) introducing said nucleic acid construct(s) into a plant cell

37. Method for ectopic expression of a gene of interest in a plant cell comprising:
a) operably linking the upstream activator sequence of an RNR1b gene to a gene of interest in a nucleic acid construct, and
b) introducing said nucleic acid construct(s) into a plant cell

38. Method according to claim 33 to 37 further comprising generating a plant from said plant cell.

39. Method for building an expression cassette that is active in the G1/S transition phase and/or in the S phase and/or in meristematic cells comprising operably linking an E2F element located in a 5' UTR or a transcribed region of a gene, as described in claims 1 to 9, to a promoter

40. Method for improving the expression of a gene of interest in the G1/S transition phase and/or in the S phase and/or in meristematic cells, and/or improving the regulation of a gene of interest in the G1/S transition phase and/or in the S phase and/or in meristematic cells comprising operably linking an E2F element located in a 5'UTR or a transcribed region of a gene, as described in claims 1 to 9, to said gene of interest.

41. Method for building a bi-directional promoter that is active in the G1/S transition phase and/or in the S phase and/or in meristematic cells comprising operably linking at least one E2F element located in a 5' UTR or a transcribed region of a gene, as described in claims 1 to 9, to a promoter.

42. Transgenic plants obtainable by any of the methods of claims 33 to 38

43. A harvestable part of a plant of claim 42

44. The harvestable part of a plant of claim 43 which is selected from the group consisting of seeds, leaves, fruits, stem cultures, rhizomes and bulbs.

45. The progeny derived from any of the plants or plant parts of any of claims 42 to 44.


SEQUENCE LISTING

<110>  Centre National de la Recherche Scientifique DAE

<120>   G1/S phase specific, s phase specific and meristem specific transcription c
ontrol
element in a transcribed region and the uses thereof

<130>  CROP-027-EP

<140>  EP 02290389.2
<141>  2002-02-18

<160>  13

<170>  PatentIn version 3.1

<210>  1
<211>  182
<212>  DNA
<213>  Nicotiana tabacum

<400>  1
attaggcggg aaaattttaa aaccctaatt tcattcttca aattaacgag ggaaattcaa      60

tacaaaattc aaaagttgct tgttatgcta tatatacaca cacacctcac tgaaatcgaa     120

gcaatccatt ccatctgaca actgtttgac gaaattcctc agtgaaagaa agaatcgaga     180

aa                                                                    182


<210>  2
<211>  443
<212>  DNA
<213>  Nicotiana tabacum

<400>  2
gtccataaaa ttttgtaagt tttttctaga ttttttgaaa atattttttc caaaaatcaa      60

aaaatgattt agaccacttt ttcaaaattt tcaaaaaatt ttattttcca actcacaaaa     120

ctgtaatatt ttttcaagtg aaatgcatgt ctaaacataa ttttaaatta caaaaatact     180

atttttcaat ttcaatttaa atattacttt tttcaaaaat tataattttt atgtcgaaat     240

gccttctaaa ttacaaatat atgaaataaa atccaatttt tttttcactt tcattaacta     300

tgtccccttt tacaattatc gcaaattata gtttccacta aaatcttaac aaagtgggcc     360

cacatccagc ttctcctcca ttctcttttt cctttttcag ccatatttga tatttacccg     420

ccaacagtca cccccgctct tct                                             443


<210>  3
<211>  625
<212>  DNA
<213>  Nicotiana tabacum

<400>  3
gtccataaaa ttttgtaagt tttttctaga ttttttgaaa atattttttc caaaaatcaa      60

aaaatgattt agaccacttt ttcaaaattt tcaaaaaatt ttattttcca actcacaaaa 120

ctgtaatatt ttttcaagtg aaatgcatgt ctaaacataa ttttaaatta caaaaatact 180

atttttcaat ttcaatttaa atattacttt tttcaaaaat tataattttt atgtcgaaat 240

gccttctaaa ttacaaatat atgaaataaa atccaatttt tttttcactt tcattaacta 300

tgtccccttt tacaattatc gcaaattata gtttccacta aaatcttaac aaagtgggcc 360

cacatccagc ttctcctcca ttctcttttt cctttttcag ccatatttga tatttacccg 420

ccaacagtca cccccgctct tctattaggc gggaaaattt taaaacccta atttcattct 480

tcaaattaac gagggaaatt caatacaaaa ttcaaaagtt gcttgttatg ctatatatac 540

acacacacct cactgaaatc gaagcaatcc attccatctg acaactgttt gacgaaattc 600

ctcagtgaaa gaaagaatcg agaaa 625

<210> 4
<211> 9
<212> DNA
<213> Nicotiana tabacum

<220>
<221> misc_feature
<222> (1)..(9)
<223> Reverse E2F control element

<400> 4
ggccccaaa 9

<210> 5
<211> 20
<212> DNA
<213> Artificial Sequence

<220>
<223> Oligonucleotide

<400> 5
ttaggcggga aaattttaaa 20

<210> 6
<211> 20
<212> DNA
<213> Artificial Sequence

<220>
<223> Oligonucleotide

<400> 6
ttatttaaag tcattttaaa 20

<210> 7
<211> 28
<212> DNA

<213>  Artificial Sequence

<220>
<223>  Oligonucleotide

<400>  7
tgccatcacg aagcttacta atatgaac                                          28


<210>  8
<211>  9
<212>  DNA
<213>  Nicotiana tabacum

<220>
<221>  misc_feature
<222>  (1)..(9)
<223>  Telo box


<400>  8
aaaacccta                                                               9


<210>  9
<211>  9
<212>  DNA
<213>  Nicotiana tabacum

<220>
<221>  misc_feature
<222>  (1)..(9)
<223>  MYB box


<400>  9
gccaacagt                                                               9


<210>  10
<211>  8
<212>  DNA
<213>  Artificial Sequence

<220>
<223>  E2F binding site - consensus sequence

<220>
<221>  misc_feature
<222>  (4)..(5)
<223>  N = C or G


<400>  10
tttnncgc                                                                8


<210>  11
<211>  8
<212>  DNA
<213>  Artificial Sequence

<220>

<223>  Reverse E2F binding site

<400>  11
gcgggaaa                                                                 8


<210>  12
<211>  6
<212>  DNA
<213>  Artificial Sequence

<220>
<223>  Telo box

<400>  12
caacag                                                                   6


<210>  13
<211>  628
<212>  DNA
<213>  Nicotiana tabacum

<400>  13
gtccataaaa ttttgtaagt tttttctaga ttttttgaaa atattttttc caaaaatcaa      60

aaaatgattt agaccacttt ttcaaaattt tcaaaaaatt ttattttcca actcacaaaa     120

ctgtaatatt ttttcaagtg aaatgcatgt ctaaacataa ttttaaatta caaaaatact     180

atttttcaat ttcaatttaa atattacttt tttcaaaaat tataattttt atgtcgaaat     240

gccttctaaa ttacaaatat atgaaataaa atccaatttt tttttcactt tcattaacta     300

tgtccccttt tacaattatc gcaaattata gtttccacta aaatcttaac aaagtgggcc     360

cacatccagc ttctcctcca ttctcttttt cctttttcag ccatatttga tatttacccg     420

ccaacagtca cccccgctct tctattaggc gggaaaattt taaaacccta atttcattct     480

tcaaattaac gagggaaatt caatacaaaa ttcaaaagtt gcttgttatg ctatatatac     540

acacacacct cactgaaatc gaagcaatcc attccatctg acaactgttt gacgaaattc     600

ctcagtgaaa gaaagaatcg agaaaatg                                        628